(19) Europäisches Patentamt
European Patent Office
Office européen des brevets

(11) Publication number: 0 080 739 A2

# (12) EUROPEAN PATENT APPLICATION

(21) Application number: 82111790.0

(22) Date of filing: 04.07.80

(51) Int. Cl.³: C 07 D 233/60

(30) Priority: 06.07.79 JP 86249/79

(43) Date of publication of application: 08.06.83 Bulletin 83/23

(84) Designated Contracting States: DE FR GB

(60) Publication number of the earlier application in accordance with Art. 76 EPC: 0 031 388

(71) Applicant: SMITHKLINE BECKMAN CORPORATION
1 Franklin Plaza
Philadelphia Pennsylvania 19101(US)

(72) Inventor: Teraji, Tsumotu
20-6, Kofudai 6-chome Toyono-cho
Toyono-gun Osaka 563-01(JP)

(72) Inventor: Nakai, Yoshiharu
7-18, Zeze 1-chome
Otsu-shi Shiga 520(JP)

(72) Inventor: Durant, Graham John
401 Knightsfield
Welwyn Garden City Herfordshire(GB)

(74) Representative: Hargreaves, Gerald Henry, Dr. et al,
SMITH KLINE & FRENCH Laboratories Limited Patent
Department Mundells
Welwyn Garden City Hertfordshire AL7 1EY(GB)

## (54) Process for preparing imidazole derivatives.

(57) Imidazole derivatives including cimetidine having histamine-$H_2$ antagonist activity and of the formula

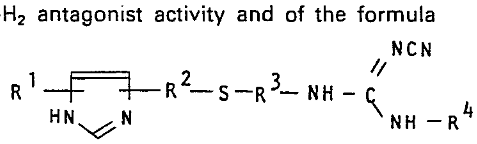

where $R^1$ and $R^4$ are each lower alkyl, and $R^2$ and $R^3$ are each lower alkylene, are prepared by reacting cyanamide with a compound of the formula

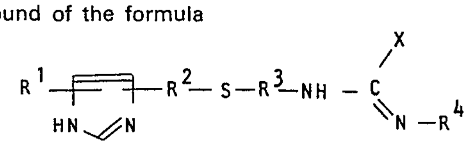

where X is halogen or a group $-S(O)_n-Z$, in which Z is hydroxy or alkyl and n is 1 or 2.

EP 0 080 739 A2

## SPECIFICATION

## PROCESS FOR PREPARING IMIDAZOLE DERIVATIVES

This invention relates to a process for preparing imidazole derivatives of the formula

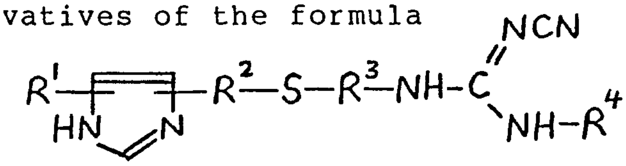

(I)

where $R^1$ and $R^4$ are each lower alkyl, and $R^2$ and $R^3$ are each lower alkylene, and their salts.

Imidazole derivatives of the formula (I) and their salts, which include cimetidine, are compounds having histamine $H_2$-antagonist activity and useful as anti-ulcer agents.

According to the invention a process for preparing an imidazole derivative of formula (I) comprises reacting cyanamide with a corresponding compound of the formula

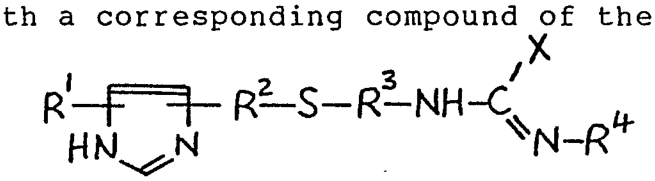

(II)

characterised in that X is halogen or a group $-S(O)_n-Z$ in which Z is hydroxy or alkyl, and n is 1 or 2.

In the starting material (II) each of $R^1$ and $R^4$ may be a straight or branched alkyl group having 1 to 6 carbon atoms such as methyl, ethyl, propyl, isopropyl, butyl, isobutyl, tert-butyl, pentyl or hexyl, and each of $R^2$ and $R^3$ may be a straight or branched alkylene group having 1 to 6 carbon atoms such as methylene, ethylene, trimethylene, tetramethylene, pentamethylene, hexamethylene, 2-methyltrimethylene or 2,2-dimethyltrimethylene. $R^2$ and $R^3$ may be the same or different from each other. When X is halogen, it may be chlorine,

bromine and iodine. When X is a group $-S(O)_n-Z$, it may be sulfino, sulfo, alkylsulfinyl having 1 to 6 carbon atoms (e.g. methylsulfinyl, ethylsulfinyl, propylsulfinyl, isopropylsulfinyl, butylsulfinyl, isobutylsulfinyl, tert-butylsulfinyl, pentylsulfinyl and hexylsulfinyl), or alkylsulfonyl having 1 to 6 carbon atoms (e.g. methylsulfonyl, ethyl-sulfonyl, propylsulfonyl, isopropylsulfonyl, butylsulfonyl, isobutylsulfonyl, tert-butylsulfonyl, pentylsulfonyl, and hexysulfonyl).

The reaction can be carried out in a conventional solvent such as methylene dichloride, acetonitrile, and dimethylformamide. The reaction temperature is not critical and the reaction is generally carried out at elevated temperature.

The cyanamide can be used as such or as an alkali metal (e.g. sodium and potassium) salt.

Where the cyanamide is used in free form, the reaction is preferably carried out in the presence of a base such as triethylamine, pyridine, N-methylaniline and 1,5-diazabicyclo[5.4.0]undec-5-ene.

Where a compound (II) in which X is sulfino or sulfo group is used, it can be in the form of a salt such as a salt of an alkali metal (e.g. sodium and potassium) or a 1,5-diazabicyclo[5.4.0]undec-5-ene (DBU) salt.

The imidazole derivative (I) produced by a process of this invention may be isolated and purified, and if desired, converted into its salt such as a hydrochloride in a conventional manner.

The starting materials of formula (II) can be prepared as described in the following Example or by similar methods, or by methods similar to those described for the sulfinyl and sulfonyl compounds in European Patent Specification 80901220.6.

The following Example illustrates the preparation of cimetidine.

EXAMPLE

A solution of N-methyl-chloroformimidoyl chloride (1.12 g), 2-(5-methylimidazol-4-ylmethylthio)-ethylamine (0.855 g) and DBU (0.988 g) in methylene dichloride (16 ml) was heated under reflux for 6 hr. To the reaction mixture containing N-methyl-N'-[2-(5-methylimidazol-4-ylmethylthio)ethyl]chloroformamidine was added a solution of cyanamide (0.84 g) and DBU (3.04 g) in methylene dichloride (16 ml). After refluxing for 15 hr, the solvent was removed and the residue was chromatographed [$SiO_2$, $CH_3CN-CH_3OH$ (4:1)]. The crude product was purified by preparative chromatography [$SiO_2$, $CH_3COOC_2H_5-CH_3OH-$ 28% aq. $NH_3$ (10:1:1)] and recrystallisation from acetonitrile to give N-cyano-N'-methyl-N"-[2-(5-methylimidazol-4-ylmethylthio)ethyl]-guanidine (0.24 g), yield 19.0%, m.p. 139-141$^{\circ}$C.

CLAIMS

1. A process for preparing an imidazole derivative of the formula

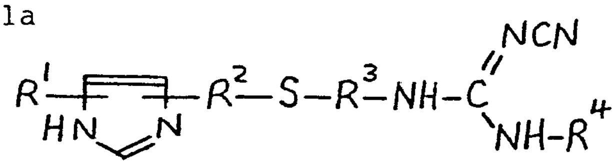

where $R^1$ and $R^4$ are each lower alkyl, and $R^2$ and $R^3$ are each lower alkylene, which comprises reacting cyanamide with a corresponding compound of the formula

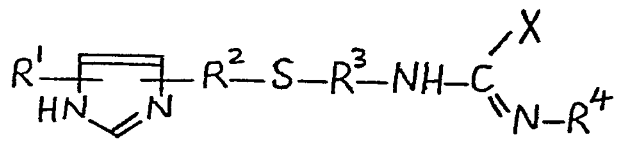

characterised in that X is halogen or a group $-S(O)_n-Z$ in which Z is hydroxy or alkyl and n is 1 or 2.

2. A process according to Claim 1, in which the imidazole derivative is cimetidine.